Europäisches Patentamt

European Patent Office

Office européen des brevets

(11) Publication number: 0 488 243 A1

# EUROPEAN PATENT APPLICATION

(21) Application number: 91120311.5

(22) Date of filing: 27.11.91

(51) Int. Cl.5: C12Q 1/70, C12Q 1/68,
//C07H21/04

(30) Priority: 30.11.90 JP 329653/90
20.02.91 JP 26477/91
21.02.91 JP 27537/91
16.07.91 JP 199879/91

(43) Date of publication of application:
03.06.92 Bulletin 92/23

(84) Designated Contracting States:
AT BE CH DE DK ES FR GB GR IT LI LU NL SE

(71) Applicant: SANWA KAGAKU KENKYUSHO CO.,
LTD.
No. 35, Higashi-sotobori-cho, Higashi-ku
Nagoya, Aichi-ken(JP)

(72) Inventor: Sawai, Kiichi, c/o Sanwa Kagaku
Kenkyusho Co. Ltd., 35, Higashi-sotobori-cho
Higashi-ku, Nagoya, Aichi-ken(JP)
Inventor: Kurono, Masayasu, c/o Sanwa
Kagaku
Kenkyusho Co. Ltd., 35, Higashi-sotobori-cho
Higashi-ku, Nagoya, Aichi-ken(JP)
Inventor: Mitani, Takahiko, c/o Sanwa Kagaku
Kenkyusho Co. Ltd., 35, Higashi-sotobori-cho
Higashi-ku, Nagoya, Aichi-ken(JP)
Inventor: Jomori, Takahito, c/o Sanwa Kagaku
Kenkyusho Co. Ltd., 35, Higashi-sotobori-cho
Higashi-ku, Nagoya, Aichi-ken(JP)
Inventor: Takahashi, Haruo, c/o Sanwa
Kagaku
Kenkyusho Co. Ltd., 35, Higashi-sotobori-cho
Higashi-ku, Nagoya, Aichi-ken(JP)
Inventor: Hayashi, Yuji, c/o Sanwa Kagaku
Kenkyusho Co. Ltd., 35, Higashi-sotobori-cho
Higashi-ku, Nagoya, Aichi-ken(JP)
Inventor: Suzuki, Eiji, c/o Sanwa Kagaku
Kenkyusho Co. Ltd., 35, Higashi-sotobori-cho
Higashi-ku, Nagoya, Aichi-ken(JP)

(74) Representative: Wächtershäuser, Günter, Dr.
Tal 29
W-8000 München 2(DE)

(54) Method of extracting virus genome from sample derived from living body infected with the virus and detecting the genome.

(57) There are described methods of extracting a virus genome from a sample derived from a living body as well as detecting the genome by measuring a product due to the genome and amplified through a polymerase chain reaction. A genomic RNA or DNA of the virus is extracted through a treatment by a proteinase in the presence of a non-ionic surface active agent. In case of the genome of RNA virus, the RNA is converted into its cDNA with use of a reverse transcriptase, prior to the polymerase chain reaction. These all reactions containing the polymerase chain reaction are able to carry out with use of only one reaction tube. A possibly existing product in a reaction solution obtained after the final polymerase chain reaction is reacted with a fluorescent substance to check the presence or absence of the product by radiating UV rays. The reaction solution can be controlled in its pH in the range of 10 to 12.

The present invention relates to a method of extracting a virus genome from a sample derived from a living body infected with the virus and detecting the genome by checking the presence or absence of a product due to the genome and amplified by a polymerase chain reaction (herein after referred to merely as "PCR").

The invention will be explained with reference to a human C (Non-A, non-B) hepatitis virus, although it shall not be limited thereto.

At the present time, the human C hepatitis occupies 90 to 95% among a family of blood transfusional hepatitises, and a positive remedy thereof has not yet been established. Therefore, there is a strong demand for developments of its diagnosing method, effective medicine, agent for treating a transfusional blood to inactivate or kill the human C hepatitis virus possively presenting therein, preventive vaccine and others.

In recent years, a search on human C hepatitis virus antigens great progresses and several reports have been issued. Namely, in Jap. Pat. Nos. Sho 62 - 249999(A) and 63 - 91328(A), there is given a statement that a single-stranded RNA having about 10000 deoxyribonucleotides may concern to the human C hepatitis virus gene. While, Michael HOUGTON in Chiron Corporation located at California, U.S.A. have made a cDNA derived from a liver tissue sample of a chimpanzee infected with the human C hepatitis into its clone, and expressed the same in Escherichia coli with λ gt11 vector transformed by the cloned DNA, to fined that a product thereof reacts with a serum sample derived from a patient infected with the human C hepatitis. They have finally confirmed that the product is a single-stranded RNA having about 9000 deoxyribonucleotides, which is similar to that for filavi virus gene, named the RNA as "HCV", and disclosed a part of nucleotide sequence for the cloned DNA, namely about 7000 deoxyribonuleotide among the about 9000 deoxyribonucleotides [EP-03 18 216(Ai)].

Further, said Chiron Corporation has developed and marketed a diagosis kit which comprises a yeast transformed by a part of the HCV gene, said yeast producing a polypeptide which behaves as an antigen reacting with an antibody in a serum sample derived from a patient infected with the human C hepatitis virus, and they said that the kit is employed to judge whether a patient was infected with HCV or not, and that the judgement can be passed with about 70% in immunological probability. At the present time, it has been said that this method is suitable a screening and diagnosis of a chronic hepatopathy due to the human C hepatitis virus and screening of transfusional blood, since the HCV antibody will appear at the time after passed an acte stadium.

With the establishment of HCV clone, a detection of human C hepatitis virus genome which utilizes the PCR has been carried out [for instance, Kaneko et al, "The Lancet", Vol. 335, page 976 (1990)]. The PCR is a technique developed by Mullis K. et al ["Methods in Enzymology", page 155, Academic Press (1987)] and for amplifying a targeting DNA gene area into several hundred thousand folds to million folds or more in a relatively short period of time, due to a specificity of primers (if a virus genome is RNA, it should be converted into DNA, prior to carry out the PCR), and thus the PCR can be utilized for establishing a high sensitive detection method, since the amplification is possible, in theoretical view point, if there is only one copy of the virus genome.

However, all of conventional methods for detecting a virus genome by amplifying the same through the PCR have disadvantages of that it is, in general, difficult to extract mRNA and RNA of the virus genome or DNA virus genome without cause any contamination, since a slight contamination by a scattering of sample containing the virus genome due to opening and/or closing of a lid of micro-tube, misoperation in pipetting, carrying over due to that the amplified targeting gene sticks by mistake to experimental appliances and hands or cloth of the man who is carrying out the PCR, or the like leads such a case detecting as "positive" on a sample which should be detected as "negative", due to that the PCR is too high sensitive detection method, and that a long period of time is required, since operations of an agarose or polyacryloamide gel electrophoresis, dyeing with use of ethidium bromide, and UV radiation to observe a product due to the virus genome and take a photograph are necessary, in subsequent to the PCR.

Namely, according to the conventional techniques, a method inclusive of a treatment with use of a protein modifying agent such as guanidine isothiocyanate, phenol, urea, chloroform or the like and another treatment with use of a surface active agent such as sodium dodecylsulfate or the like are carried out for extracting and isolating the virus genome, but such a method requires steps of a centrifugal treatment to remove a protein, collecting a solution layer which contains the virus genome to transfer the same into another microtube, adding an alcohol to cause the virus genome a precipitation thereof for removing the treating agents, and drying the virus genome. Such operations require a long period of time and is not suitable to handle a large number of samples, and increase a probability of generation of contamination. Further, the method has disadvantages of that a recovery of virus genome becomes low (less than 50%) due to the precipitation with use of the alcohol, when an amount of the virus genome in the sample is very small, so that a quantitative yield can not be obtained, and that when the virus genome to be detected is RNA, the dried precipitate is once dissolved and then the RNA should be converted into its cDNA with use of a reverse transcriptase.

On the grounds as referred to, the method for detecting a product due to the virus genome amplified by utilizing the PCR to diagnose the human C hepatitis virus has not widely been employed as one of clinical examinations, in spite of that usefulness thereof had been accepted.

First object of the invention is to provide a method of extracting a virus genome from a sample derived from a living body, for instance from serum and plasma derived from a patient infected with the virus, and more particularly infected with human C hepatitis virus, with simple operations, short in required period of time and without cause any contamination.

Second object of the invention is to provide a novel method of detecting a virus genome through a measurement of a product due to the virus genome and amplified through a PCR.

Third object of the invention is to provide a method of detecting a presence or absence of a PCR product, by visual observation.

Final and fourth object of the invention is to provide a method of detecting a virus genome, and more particularly human C hepatitis virus genome, by carrying out a PCR with use of a solution of the extract given in the above Item on the first object, which method is easy in operation, is required short period of time, and provides an accurate result without cause any contamination.

The inventors have energetically studied and investigated a method of novel extraction of the virus genome from a sample derived from a living body to find that if the sample is treated with a proteinase in the presence of a non-ionic surface active agent, the resulting sample can be employed as that for subjecting to the PCR, so that concerned problems encountered to the prior arts can be overcome to attain the first object. Namely, a troublesome removal treatment of denaturated or modified protein and precipitating operation with use of an alcohol, which are required for the conventional methods can be omitted, if the method according to the invention is carried out by limiting an amount of the sample to 1 - 20$\mu$ l in case of serum or plasma sample, or 10mg or more less in case of tissue sample. Further, the method according to the invention provides additional advantages of that no exchange of reaction tube is required to exclude or reduce a chance of undesired contamination of the sample, and that the extraction of virus genome, a possible transcription reaction from RNA to its cDNA and the PCR can be carried out in a single and only one reaction tube.

In the extraction method according to the invention, a serum, plasma, exenterated tissue or the like can be used as the sample, Nonidit P-40 (trademark) or the like can be used as the non-ionic surface active agent, and proteinase K or the like can be employed as the proteinase.

According to the invention, the second object can be attained by a method, wherein a product amplified by the PCR is reacted with a fluorescent substance having an affinity to DNA. According to this method, 100 samples can be measured by about 1 hour and this means that a required time is shortened to 1/6, in comparison with the conventional detection method utilizing an electrophoresis.

As the fluorescent substance, mitoramycin or the like antibiotics; acrydine orange, ethidium bromide, 4',6'-diamidino-2-phenylindole or the like coloring matter for dyeing nucleus; and quinacrine mustard or the like anti-tumor agent can be employed. Among them, it is preferable to use mitoramycin or ethidium bromide, in view of the level of its affinity to DNA.

The third object can be attained by adding a fluorescent substance having affinity to DNA to a solution including a possible product amplified by the PCR and accommodated in a reaction tube, without taking out the solution from the reaction tube, adjusting or controlling pH of the solution, and radiating a light with specific wave-length to visually observe a presence or absence of the fluorescence. As the fluorescent substance, As the fluorescent substance, those as referred to in the preceding paragraph can be used, but ethidium bromide is most preferable. In this method, the pH control is carried out to suppress an intensity of non-specific fluorescence on "negative" sample, which shall be issued due to primers employed for the PCR. Namely, the fluorescence due to the primers in the "negative" sample can not be sensitized with the visual observation, when pH of the reaction solution is kept in a range of 10 - 12. While, under the same pH condition, a DNA existing in the "positive" sample and amplified by the PCR shall form double-stranded DNA which reacts with specificity with the fluorescent substance to issue fluorescence, so that the "negative" and "positive" samples can be accurately distinguished from each other through the visual observation.

The fourth and final object can be attained by treating a sample derived from a living body, with a proteinase in the presence of a non-ionic surface active agent; subjecting the resulting extract solution to a first polymerase chain reaction as it is, when a virus genome possibly existing in the extract solution is DNA, by adding to the extract solution a reacting solution of a set of primers and a Taq polymerase but when the virus genome possibly existing in the extract solution is RNA, after converted the RNA into its cDNA with use of a reverse transcriptase, and adding to the resulting solution a Taq polymerase; carrying out a second polymerase chain reaction on the resulting reaction solution by adding thereto a solution which comprises another set of primers and a Taq polymerase; carrying out an electrophoresis to check the presence or absence of a band of predetermined base pair length corresponding to a product due to the virus genome to be detected and amplified through the polymerase chain reactions.

Following sets of primers can be listed.

For the first PCR :

5'-GAGTGCGCCTCACACCTTCCTTACATCGAACAAGGA-3'

and

5'-ATCCCGCTGATGAAGTTCCACATGTGCTTC-3',

For the second PCR :

5'-CTTCCTTACATCGAACAAGGA-3'

and

5'-TTCCACATGTGCTTCGCCCAG-3',

or

For the first PCR :

5'-GAGTGCGCCTCACACCTTCCTTACATCGAA-3'

and

5'-AAGCCTGCTAGATACTGTATCCCGC-3',

and

For the second PCR :

5'-CTTCCTTACATCGAACAAGGA-3'

and

5'-TTCCACATGTGCTTCGCCCAG-3'.

Each of these methods is excellent in detection sensitivity of about 10 folds, in comparison with that using the conventional detection method, wherein the virus genome is extracted by the method using guanidine isothiocyanate and described in "Analytical Biochemistry", Vol. 162, pages 156 - 159 (1987) and a product due to the virus genome and amplified by the PCRs is detected.

The methods according to the invention can be modified by changing operations subsequent to the second PCR to a detection of fluorescent intensity as described hereinbefore on attainment of the second and third objects.

The invention will now be further explained in more detail and concretely with reference to Examples, Reference Example and Test Examples, which shall refer to drawings, wherein

Fig. 1 is patterns showing results of electrophoresis after polymerase chain reactions with use of plasma samples derived from HCV patients to check a concentration of proteinase required for extracting a human C hepatitis virus (HCV) genome;

Fig. 2 is patterns showing results of electrophoresis after polymerase chain reactions as to extractions extracted according to the method of invention and conventional method to check a difference in detection sensitivity therebetween;

Fig. 3A is patterns showing results of electrophoresis after polymerase chain reactions, on HCV genome extracted by the method of invention from $10\mu$ l of plasma samples derived from 12 HCV patients to detect a product due to HCV genome;

Fig. 3B is patterns similar to that in Fig. 3A, but an extraction was carried out according to a conventional manner with use of $100\mu$ l of plasma samples;

Fig. 4 is patterns showing results of electrophoresis as described in preamble part in Example 4;

Fig. 5 is trace of photographed images which have taken to confirm visual observations carried out after completion of polymerase chain reactions in a reaction tube accommodating each serum sample derived from 10 HCV patients and 10 normal persons and 2 sets of primers, adding the reaction solution comprising a fluorescent substance of ethidium bromide and a buffer for pH control, radiating UV rays in a dark place to check the presence or absence of fluorescence due to a product by HCV genome; and

Fig. 6 is a graph showing results measured with use of a fluorometer at exciting wave-length of 312nm and detecting wave-length of 590nm on the fluorescent intensities which are shown in Fig. 5.

The Examples are directed to a detection of HCV only, but it is to be noted the the method according to the invention can be applied for detection of various RNA and DNA virus genomes by selecting nucleotide sequences on each set of primers to be used for the PCR, for instance AIDS (Acquired Immunodeficiency Syndrome) can be carried out by extracting RNA of a virus genome thereon with use as a sample of lymphoblast cells or serum obtained from a patient infected with HIV (human Immunodeficiency Virus), in accordance with the method similar to that as described in the following Example(s), preparing a DNA complementary to the RNA, carrying out a PCR(s) with use of a set of primers, each of which has a nucleotide sequence encoding a part of the virus gene, and detecting of an amplified DNA.

Example 1 (Extraction of virus genome from sample derived from living human body)

To a microtube with an inner volume of 0.5ml accommodating $10\mu$ l of a plasma obtained from a patient infected with HCV, $1\mu$ l of a solvent [consisting of 500mM trishydroxyaminomethanechloride buffer (pH : 8.0), 250mM potassium chloride, 30mM magnesium chloride, 0.5% nonidit P-40 and 10mg/ml of proteinase K] was added to mix the same. The mixture was incubated for 1 hour at 55°C to obtain an extract solution of virus genome.

In the case of that an amount of the sample plasma is less than $10\mu$ l, a volume of the sample is made to $10\mu$ l by adding a solution similar to the solvent excepting that it includes none of the nonidit P-40 and proteinase K, and then the resulting solution shall be mixed with the solvent to incubate the same as above. And, in case of that a tissue sample is used, $10\mu$ l of a solution similar to the solvent excepting that it includes none of the nonidit P-40 and proteinase K were added per 10mg of the tissue, the solvent was added thereto to mix the same, and then the incubation was carried out similar to the above.

Example 2 (Conversion of RNA into DNA and amplification of DNA by PCR)

HCV virus which will be existing in the extract solution obtained by Example 1 is RNA and thus the RNA should be converted into a DNA prior to carrying out the PCR. To $11\mu$ l of the virus genome extract solution obtained in Example 1, therefore, $1\mu$ l of a solution containing 20pM of 2 primers (as described later) was added, and the mixture was incubated for 15 minutes at 99°C to eliminate the activity of proteinase K and to anneal the primer and virus RNA. To the heat treated solution, $13\mu$ l of a solution [50mM trishydroxyaminomethane-hydrochloride buffer (pH : 8.0), 25mM potassium chloride, 3mM magnesium chloride, each $250\mu$ M of 4 deoxyribonucleotides (dATP, dDIP, dCTP and dTTP), 2.5mM dithiothreitol and 40 units of RNasin] containing 14 units of a reverse transcriptase was added and the resulting mixture was incuvated for 1 hour at 45°C to prepare a complementary DNA. To the reaction solution containing said RNA and its cDNA, $50\mu$ l of a solution [50mM trishydroxyaminomethane-hydrochloride buffer (pH : 8.0), 50mM potassium chloride, 1.5mM magnesium chloride and 0.01% gelatin] containing 5 units of Taq polymerase was added to carry out a first PCR which is composed from 40 cycles and each cycle consists of thermal denaturation step for 1 minute at 94°C, annealing step for 1.5 minutes at 55 °C and elongational reaction step for 1 minute at 72°C. Thereafter, to the first PCR solution containing said amplified DNA, 50μ l of a solution [10mM trishydroxyaminomethane-hydrochloride buffer (pH : 8.0), 50mM potassium chloride, 1.5mM magnesium chloride, and each 250μ M of 4 deoxyribonucleotides (dATP, dDIP, dCTP and dTTP)] containing each 20pM of 2 primers (as described later) and 5 units of a Taq polymerase was added to carry out a second PCR similar to the first PCR in system but composed from 30 cycles.

The resulting reaction product was subjected to an electrophoresis and dyed with ethidium bromide to confirm the presence of a band with a predetermined base pair length (about 180bp) corresponding to a product due to HCV genome.

In the Sequence Listing as given later, 5 cDNA sequences of HCVs cloned by Kobayashi, K. et al inclusive one of the present inventors and disclosed in Jap. Pat. No. Hei 3 - 198777(A) [USSN 633852, EP-0 435 229(A1)], which has been assigned to present assignee company. are shown as Sequence Nos. 1 to 5. Parts of the cDNA sequence in Sequence No. 1 were employed as the set of primers for the first and second PCRs. Namely, nucleotide sequences of

5'-GAGTGCGCCTCACACCTTCCTTACATCGAACAAGGA-3'

which includes therein nucleotides in 22nd to 57th positions, and

5'-ATCCCGCTGATGAAGTTCCACATGTGCTTC-3'

which includes therein nucleotides complementary to those in 200th to 179th positions were selected for the first PCR, and nucleotide sequences of

5'-CTTCCTTACATCGAACAAGGA-3'

which includes therein nucleotides in 37th to 57th positions and

5'-TTCCACATGTGCTTCGCCCAG-3'

which includes therein nucleotides complementary to those in 194th to 174th positions were selected for the second PCR.

Example 3

Operations as described in Example 2 were repeated in same condition but with use of following sets of primers, each of which is a part of nucleotide sequence of Sequence No. 1 in the Sequence Listing given later, were selected for the PCR.

For the first PCR :

5'-GAGTGCGCCTCACACCTTCCTTACATCGAA-3'

which is nucleotides in 22nd to 51st positions and

5'-AAGCCTGCTAGATACTGTATCCCGC-3'

which is nucleotides complementary to those in 227th to 203rd positions.

For the second PCR :

5'-CTTCCTTACATCGAACAAGGA-3'

which is nucleotides in 37th to 57th positions and

$$5\text{'}-TTCCACATGTGCTTCGCCCAG-3\text{'}$$

which is nucleotides complementary to those in 194th to 174th positions.

According to this Example, the base pair length corresponding to a HCV genome product was about 160bp.

Test Example 1 (Concentration of proteinase K required for extracting HCV genome)

For determining a range of concentration required for extracting HCV genome, a concentration of proteinase K in the solvent of $1\mu$ l was changed in various value. A product by the virus genome was detected after the PCRs which were carried out with use of each $10\mu$ l of plasma derived from a patient infected with HCV and in accordance with the manner as described in Example 2.

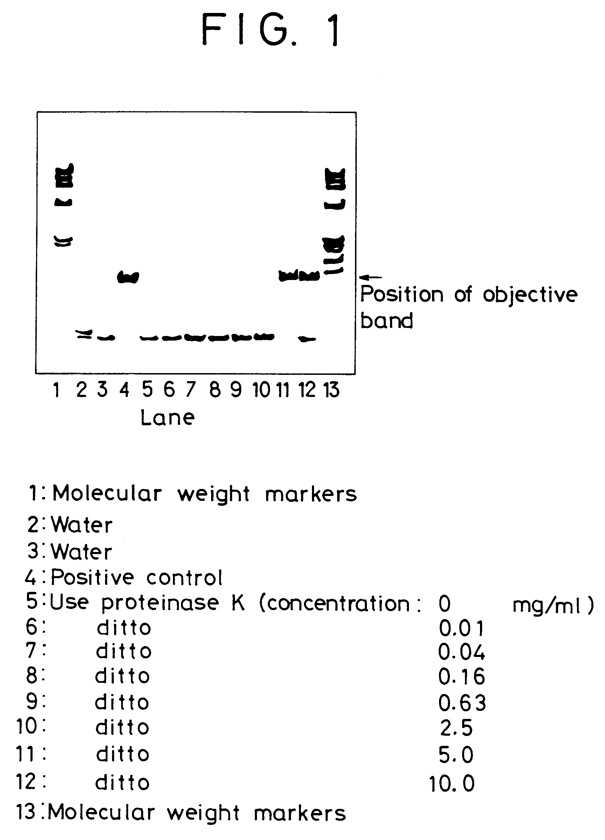

Results are shown in Fig. 1. As seen from the Figure, it has been found that an objective band can be detected, when the concentration of proteinase K is 5mg/ml or more.

Similar results have also been obtained, when the above operations was repeated, excepting that 2 sets of the primers as described in Example 3 are employed for the PCRs.

Comparative Example (Comparison in detection sensitivity between method of invention and prior art method)

A product due to HCV genome was detected after the the PCRs which were carried out with use of plasma derived from single patient infected with HCV and in accordance with the manner as described in Example 2, to compare a detection sensitivity in case where the extraction method according to the invention as described in Example 1 and a conventional method using guanidine isothiocyanate and as described in "Analytical Biochemistry", Vol. 162, pages 156 - 159 (1987) were employed.

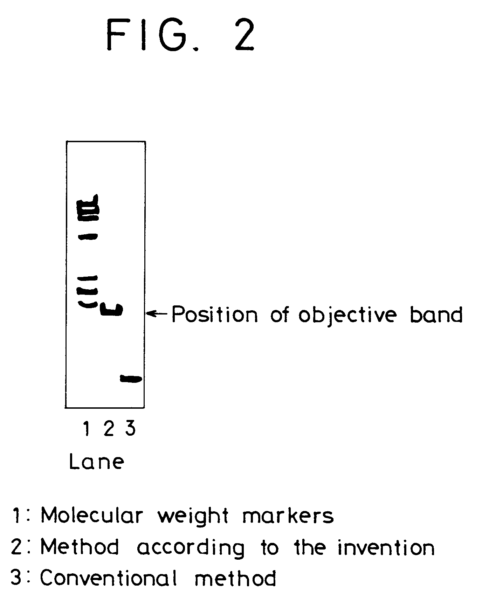

Fig. 2 is patterns showing results of electrophoresis, when the detection operations were carried out with use of the plasma sample of $10\mu$ l. As seen from the Figure, a desired objective band due to a product of HCV genome can be detected, only when the extraction was carried out in accordance with the method of invention. In connection with this, it has been found that the plasma sample is required in the amount of $100\mu$ l or more to make possible the detection, when the conventional extraction method is employed. This means that the method according to the invention is excellent in detection sensitivity of about 10 folds than the conventional method.

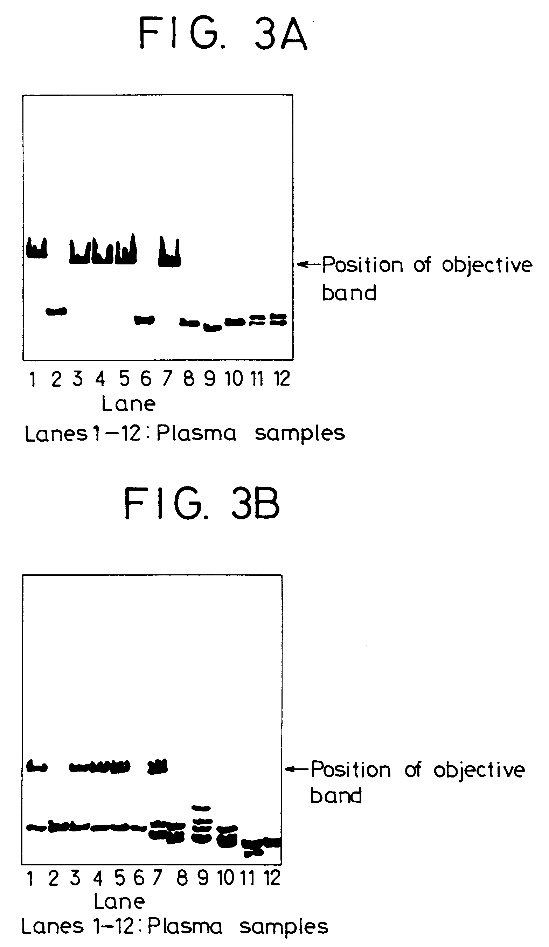

Fig. 3A is patterns showing results of electrophoresis, when the extraction of the HCV genome from plasma (each $10\mu$ l) of 12 hepatitic patient was carried out in accordance with the method as described in Example 1, amplifying the genome through the PCRs and then subjecting to electrophoresis in accordance with the method as described in Example 2. While, Fig. 3B is patterns showing results of electrophoresis similar to those in Fig. 3A, excepting that the extraction of virus genome was carried out in accordance with the conventional method as described in said literature of "Analytical Biochemistry", Vol. 162, pages 156 - 159 (1987) and with use of the plasma in each $100\mu$ l. In Figs. 3A and 3B, the same lane number is given, in case of that the plasma derived from same patient was employed.

As apparently seen from the Figures, a band due to a product of virus genome was detected in lane numbers 1, 3, 4, 5 and 7 in both cases. Therefore, it can be said that the method according to the invention is equal or more excellent in reliability, although an amount of the plasma used for the extraction is 1/10, in comparison with the conventional method.

Similar results have also been obtained, when the above operations was repeated, excepting that 2 sets of the primers as described in Example 3 were employed for the PCRs.

Example 4 (Detection of product due to HCV genome and amplified through PCR, with use of mitoramycin)

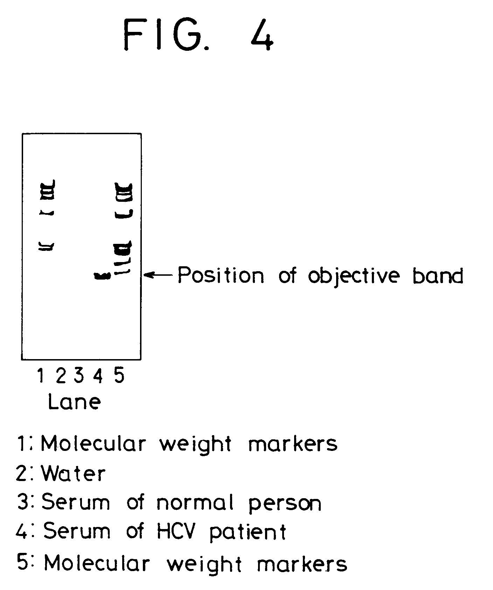

The procedure as described in Example 1 was repeated, excepting that 2 serum samples, one derived from HCV patient and the other from normal person were employed. Then, the procedure as described in Example 2 was repeated to detect the presence or absence of a due to HCV genome, through electrophoresis. Results are shown in Fig. 4. As seen from the Figure, a band due to the product of HCV genome and amplified through the PCRs was detected at the estimated position of about 160 base pairs length, in case of the sample of HCV patient serum. While, no band was detected, in case of the sample of normal person serum.

Therefore, both of the samples are used to react with a fluorescent substance of mitoramycin, as stated below.

To 20$\mu$ l of a solution which has been completed the second PCR, 10$\mu$ l of a 200$\mu$ g/ml of mitoramycin in a solution of 370$\mu$ l of distilled water and 300mM of magnesium chloride to measure an intensity of fluorescence at excitation wave-length of 440nm and detection wave-length of 540nm. Results are shown in following Table 1.

Table 1

| | Intensity of fluorescence |
|---|---|
| Serum of HCV patient (positive) | 1.5 ± 0.2 |
| Serum of normal person (negative) | 0.4 ± 0.1 |
| Blank (without use any serum) | 0.4 ± 0.1 |

The intensity of fluorescence on the sample of HCV patient serum is about 3 - 4 folds, in comparison with that on negative sample and thus it can be judged by setting an fluorescent intensity value on the blank to a value to be cut off that a sample showing the fluorescent intensity higher than cut off value includes a product due to HCV and amplified through the PCRs.

Example 5 (Detection of product due to HCV genome and amplified through PCR, with use of ethidium chloride)

This test was carried out with use of the positive and negative samples as described in the preamble part of Example 4.

To 20$\mu$ l of a solution which has been completed the second PCR, 370$\mu$ l of distilled water and 10$\mu$ l of 20$\mu$ l/ml of ethidium bromide in distilled water were added to measure an intensity of fluorescence at excitation wave-length of 260nm and detection wave-length of 450nm. Results are shown in following Table 2.

Table 2

| | Intensity of fluorescence |
|---|---|
| Serum of HCV patient (positive) | 12.1 ± 1.3 |
| Serum of normal person (negative) | 5.7 ± 0.8 |
| Blank (without use any serum) | 5.4 ± 0.5 |

The intensity of fluorescence on the sample of HCV patient serum is about 2 folds, in comparison with that on negative sample and thus it can be judged by setting an fluorescent intensity value on the blank to a value to be cut off that a sample showing the fluorescent intensity higher than cut off value includes a product due to HCV genome and amplified through the PCRs.

Example 6 (Detection of HCV genome)

The procedure as described in Example 1 was repeated, excepting that 10 serum samples derived from HCV patients and 10 serum samples derived from normal persons were employed. Then, the procedures as described in Example 2 was repeated to find that a band due to a product of HCV genome and amplified through the PCRs was detected at the estimated position of about 160 base pairs length, in all cases of the sample on the HCV patient serum, and that no such a band was detected, in all case of the sample on the normal person serum.

Therefore, following test was carried out with use of those 20 samples, since those can be recognized as suitable for the test.

To a reaction tube, in which each sample was subjected to PCR twice as described in Example 2. 20 $\mu$ l of 1M glycine-NaOH buffer (pH : 12) and 8$\mu$ l of 10$\mu$ g/ml ethidium bromide solution were directly added to control pH to 11.1. To the reaction tube accommodating the pH controlled reaction solution, UV rays were radiated in a dark place with use of a transilluminator or UV lamp to visually observe the presence or absence of fluorescence.

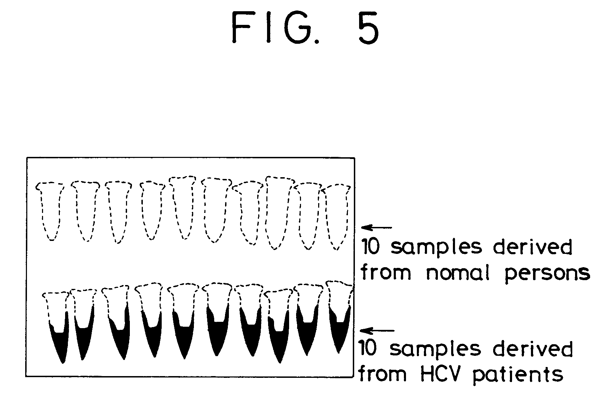

Results are shown in Fig. 5. As apparently seen from the Figure, a clear and distinct fluorescence can be recognized on all of the samples derived from HCV patient, but no fluorescence can be recognized on all of the samples derived from normal person.

To check an intensity of the fluorescence generated from the reaction solution in each reaction tube, the reaction solution was taken out from the tube to measure the intensity of the fluorescence by setting a fluorometer to excitation wave-length of 312nm and detection wave-length of 590nm.

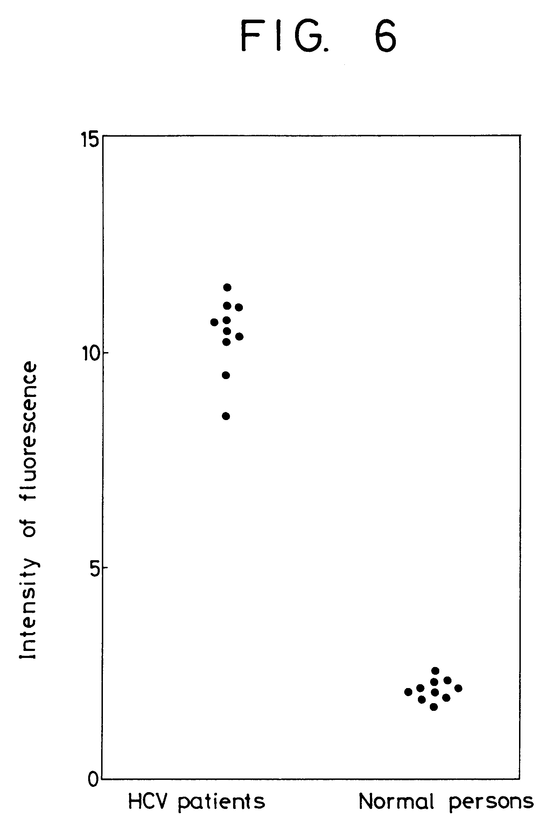

Results are shown in Fig. 6. As seen from the Figure, a value of fluorescent intensity of reaction solution on the all samples of normal persons is quite low. On contrary thereto, a value on the samples of HCV patients is about 5 fold, in comparison with that on the samples of normal persons and this supports the results of visual observation, which means that the visual observation is possible to provide a sufficient reliability to make taking of troublesome photograph unnecessary.

Test Example 2

An intensity of fluorescence was measured as described in Example 6, as to a control sample, wherein ethidium bromide was added to the reaction tube after the PCRs but pH control was not carried out as well as a test sample, wherein ethidium bromide was added and pH control was carried out. to compare the same.

Results are shown in following Table 3 (in the table, the value is given as mean value on 3 samples). From the Table, it can be seen that in case of no pH control, a judgement under visual observation whether "positive" or "negative" is difficult, since the fluorescent intensity of the reaction solution on the control serum sample derived from the normal persons reaches to the value of about 10 and there is no remarkable difference with that on the test serum sample derived from HCV patients, that by controlling the pH according to the invention, the fluorescent intensity on the control sample becomes about 1/4 to make almost insensible with visual observation, and that although the fluorescent intensity on the test sample becomes low by controlling the pH, a judgement whether the sample is "negative" or "positive" can easily be passed, since a level of the reduction is 1/2.5 and there is a difference of about 5 folds, in comparison with the fluorescent intensity on the control sample.

Table 3

| Samples | Intensity of fluorescent | |
|---|---|---|
| | No pH control | pH controlled |
| Test | 25.6 ± 3.2 | 10.4 ± 1.1 |
| Control | 9.8 ± 1.7 | 2.3 ± 0.2 |
| Blank (no serum is added) | 6.8 ± 0.9 | 1.0 ± 0.1 |

## SEQUENCE LISTING

SEQ ID NO : 1

SEQUENCE TYPE : Nucleotide with corresponding protein

SEQUENCE LENGTH : 240 base pairs

STRANDNESS : single

TOPOLOGY : linear

MOLECULE TYPE : genomic DNA

ORIGINAL SOURCE

ORGANISM : human

PROPERTIES : human C hepatitis virus gene

```
          10        20        30        40        50        60
CAGGAGTTTGATGAGATGGAGGAGTGCGCCTCACACCTTCCTTACATCGAACAAGGAATG
GlnGluPheAspGluMetGluGluCysAlaSerHisLeuProTyrIleGluGlnGlyMet
               5                10                15                20


          70        80        90       100       110       120
CAGCTCGCCGAGCAATTCAAGCAGAAGGCGCTCGGGCTGCTGCAAACAGCCTCCAAGCAA
GlnLeuAlaGluGlnPheLysGlnLysAlaLeuGlyLeuLeuGlnThrAlaSerLysGln
               25               30               35               40


         130       140       150       160       170       180
GCGGAGGCTGCTGCTCCCGTGGTAGAGTCCAAGTGGCAAGCCCTTGAGGCCTTCTGGGCG
AlaGluAlaAlaAlaProValValGluSerLysTrpGlnAlaLeuGluAlaPheTrpAla
               45               50               55               60


         190       200       210       220       230       240
AAGCACATGTGGAACTTCATCAGCGGGATACAGTATCTAGCAGGCTTGTCCACCCTGCCT
LysHisMetTrpAsnPheIleSerGlyIleGlnTyrLeuAlaGlyLeuSerThrLeuPro
               65               70               75               80
```

SEQ ID NO : 2
SEQUENCE TYPE : Nucleotide with corresponding protein
SEQUENCE LENGTH : 240 base pairs

STRANDNESS : single
TOPOLOGY : linear
MOLECULE TYPE : genomic DNA

ORIGINAL SOURCE
ORGANISM : human

PROPERTIES : human C hepatitis virus gene

```
            10        20        30        40        50        60
CGGGAGTTCGATGAGATGGAGGAGTGCGCTTCACACCTCCCTTACATCGAACAGGGAATG
ArgGluPheAspGluMetGluGluCysAlaSerHisLeuProTyrIleGluGlnGlyMet
                5                10                15                20

            70        80        90       100       110       120
CAGCTCGCCGAGCAATTCAAGCAGAAGGCGCTCGGGCTGTTGCAGACAGCCACCAAGCAA
GlnLeuAlaGluGlnPheLysGlnLysAlaLeuGlyLeuLeuGlnThrAlaThrLysGln
                25                30                35                40

           130       140       150       160       170       180
GCGGAGGCTGCAGCTCCCGTGGTCGAGTCTAAATGGCGGGCTCTTGAGACCTTCTGGGCG
AlaGluAlaAlaAlaProValValGluSerLysTrpArgAlaLeuGluThrPheTrpAla
                45                50                55                60

           190       200       210       220       230       240
AAGCACATGTGGAATTTCATCAGCGGGATACAGTACCTAGCAGGCTTGTCCACTCTGCCT
LysHisMetTrpAsnPheIleSerGlyIleGlnTyrLeuAlaGlyLeuSerThrLeuPro
                65                70                75                80
```

SEQ ID NO : 3

SEQUENCE TYPE : Nucleotide with corresponding protein

SEQUENCE LENGTH : 240 base pairs

STRANDNESS : single

TOPOLOGY : linear

MOLECULE TYPE : genomic DNA

ORIGINAL SOURCE

ORGANISM : human

PROPERTIES : human C hepatitis virus gene

```
            10        20        30        40        50        60
CAAGAGTTCGACGAAATGGAAGAGTGCGCCTCACACCTTCCTTACATCGAACAGGGAATG
GlnGluPheAspGluMetGluGluCysAlaSerHisLeuProTyrIleGluGlnGlyMet
             5        10        15        20

            70        80        90       100       110       120
CAGCTCGCCGAGCAATTCAAGCAGAAAGCGCTCGGTTTGCTGCAGACAGCTACCAAGCAA
GlnLeuAlaGluGlnPheLysGlnLysAlaLeuGlyLeuLeuGlnThrAlaThrLysGln
            25        30        35        40

           130       140       150       160       170       180
GCGGAGGCTGCAGCCCCCGTGGTGGAGTCCAAGTGGCGGGCCCTTGAGACTTTCTGGGCG
AlaGluAlaAlaAlaProValValGluSerLysTrpArgAlaLeuGluThrPheTrpAla
            45        50        55        60

           190       200       210       220       230       240
AAGCACATGTGGAATTTCATCAGCGGGATACAATACTTAGCAGGCTTATCCACTTTGCCT
LysHisMetTrpAsnPheIleSerGlyIleGlnTyrLeuAlaGlyLeuSerThrLeuPro
            65        70        75        80
```

12

SEQ ID NO : 4

SEQUENCE TYPE : Nucleotide with corresponding protein

SEQUENCE LENGTH : 240 base pairs


STRANDNESS : single

TOPOLOGY : linear

MOLECULE TYPE : genomic DNA


ORIGINAL SOURCE

ORGANISM : human


PROPERTIES : human C hepatitis virus gene


```
               10        20        30        40        50        60
CCGGCGTTCGATGAAATGGAAGAGTGTGCCTCACACCTCCCTTACATCGAACAAGGAATG
ProAlaPheAspGluMetGluGluCysAlaSerHisLeuProTyrIleGluGlnGlyMet
               5                 10                15                20


               70        80        90       100       110       120
CGGCTCGCCGAACAATTCAAGCAGAAGGCGCTCGGGTTGCTGCAAACGGCCACCAAGCAA
GlnLeuAlaGluGlnPheLysGlnLysAlaLeuGlyLeuLeuGlnThrAlaThrLysGln
              25                30                35                40


              130       140       150       160       170       180
GCGGAGGCTGCCGCTCCCGTGGTGGAGTCCAAGTGGCGTACCCTTGAGGCCTTCTGGGCG
AlaGluAlaAlaAlaProValValGluSerLysTrpArgThrLeuGluAlaPheTrpAla
              45                50                55                60


              190       200       210       220       230       240
AAGCACATGTGGAATTTCATCAGCGGGATACAATACCTAGCAGGCTTGTCCACTCTGCCT
LysHisMetTrpAsnPheIleSerGlyIleGlnTyrLeuAlaGlyLeuSerThrLeuPro
              65                70                75                80
```

13

SEQ ID NO : 5

SEQUENCE TYPE : Nucleotide with corresponding protein

SEQUENCE LENGTH : 240 base pairs

STRANDNESS : single

TOPOLOGY : linear

MOLECULE TYPE : genomic DNA

ORIGINAL SOURCE

ORGANISM : human

PROPERTIES : human C hepatitis virus gene

```
              10         20         30         40         50         60
CAGGAGTTTGATGAGATGGAGGAGTGCGCCTCACACCTTCCTTACATCGAACAAGGAATG
GlnGluPheAspGluMetGluGluCysAlaSerHisLeuProTyrIleGluGlnGlyMet
                         5                    10                   15                   20

              70         80         90        100        110        120
CAGCTCGCCGAGCAATTCAAGCAGAAGGCGCTCGGGCTGCTGCAAACGGCCTCCAAGCAA
GlnLeuAlaGluGlnPheLysGlnLysAlaLeuGlyLeuLeuGlnThrAlaSerLysGln
                        25                   30                   35                   40

             130        140        150        160        170        180
GCGGAGGCTGCTGCTCCCGTGGTAGAGTCCAAGTGGCAAGCCCTTGAGGCCTTCTGGGCG
AlaGluAlaAlaAlaProValValGluSerLysTrpGlnAlaLeuGluAlaPheTrpAla
                        45                   50                   55                   60

             190        200        210        220        230        240
AAGCACATGTGGAATCTCATCAGCGGGATACAGTATCTAGCAGGCTTGTCCACCCTGCCT
LysHisMetTrpAsnLeuIleSerGlyIleGlnTyrLeuAlaGlyLeuSerThrLeuPro
                        65                   70                   75                   80
```

14

**Claims**

1. A method of extracting a virus genome, which comprises a step of treating a sample derived from a living body, with use of a proteinase in the presence of a non-ionic surface active agent.

2. A method as claimed in Claim 1, wherein said sample is selected from the group consisting of serum, plasma and exenterated tissue.

3. A method as claimed in Claim 1, wherein said proteinase is proteinase K.

4. A method as claimed in Claim 1, wherein said non-ionic surface active agent is nonidit P-40.

5. A method as claimed in Claim 1, wherein said virus genome is a human C hepatitis virus genome.

6. A method of detecting a virus genome, which comprises steps of treating a sample derived from a living body, with a proteinase in the presence of a non-ionic surface active agent; subjecting the resulting extract solution to a first polymerase chain reaction as it is, when a virus genome possibly existing in the extract solution is DNA, by adding to the extract solution a reacting solution of a set of primers and a Taq polymerase but when the virus genome possibly existing in the extract solution is RNA, after converted the RNA into its cDNA with use of a reverse transcriptase, and adding to the resulting solution a Taq polymerase; carrying out a second polymerase chain reaction on the resulting reaction solution by adding thereto a solution which comprises another set of primers and a Taq polymerase; carrying out an electrophoresis to check a presence or absence of a band of predetermined base pair length corresponding to a product due to the virus genome to be detected.

7. A method as claimed in Claim 6, wherein all procedures are carried out with use of only one reaction tube.

8. A method as claimed in Claim 6, wherein an amount of said sample is 1 - 20$\mu$ l, in case of that the sample is selected from the group of serum and plasma, and an amount of said sample is not exceeding 10mg, in case of that the sample is an exenterated tissue.

9. A method as claimed in Claim 6, wherein said set of primers detecting HCV for the first polymerase chain reaction are

$$5'-GAGTGCGCCTCACACCTTCCTTACATCGAACAAGGA-3'$$

and

$$5'-ATCCCGCTGATGAAGTTCCACATGTGCTTC-3'$$

and said set of primers for the second polymerase chain reaction are

$$5'-CTTCCTTACATCGAACAAGGA-3'$$

and

$$5'-TTCCACATGTGCTTCGCCCAG-3'.$$

10. A method as claimed in Claim 6, wherein said set of primers detecting HCV for the first polymerase chain reaction are

5'-GAGTGCGCCTCACACCTTCCTTACATCGAA-3'

and

5'-AAGCCTGCTAGATACTGTATCCCGC-3'

and said set of primers for the second polymerase chain reaction are

5'-CTTCCTTACATCGAACAAGGA-3'

and

5'-TTCCACATGTGCTTCGCCCAG-3'.

**11.** A method of detecting a virus genome, which comprises steps of treating a sample derived from a living body, with a proteinase in the presence of a non-ionic surface active agent; subjecting the resulting extract solution to a first polymerase chain reaction as it is, when a virus genome possibly existing in the extract solution is DNA, by adding to the extract solution a reacting solution of a set of primers and a Taq polymerase but when the virus genome possibly existing in the extract solution is RNA, after converted the RNA into its cDNA with use of a reverse transcriptase, and adding to the resulting solution a Taq polymerase; carrying out a second polymerase chain reaction on the resulting reaction solution by adding thereto a solution which comprises another set of primers and a Taq polymerase; and reacting a product due to the virus genome and amplified through the polymerase chain reaction with a fluorescent substance having an affinity to DNA to check the presence or absence of the virus genome based on the fluorescence.

**12.** A method as claimed in Claim 11, wherein all procedures are carried out with use of only one reaction tube.

**13.** A method as claimed in Claim 11, wherein said fluorescent substance is selected from the group consisting of a fluorescent antibiotics, coloring matter for dyeing nucleus and fluorescent anti-tumor agent.

**14.** A method as claimed in Claim 11, wherein a pH control is carried out subsequent to the second polymerase chain reaction.

**15.** A method as claimed in Claim 14, wherein pH of the reaction solution is adjusted in a range of 10 - 12.

# F I G. 1

→ Position of objective band

1 2 3 4 5 6 7 8 9 10 11 12 13
Lane

1: Molecular weight markers

2: Water

3: Water

4: Positive control

5: Use proteinase K (concentration: 0        mg/ml )

| 6: | ditto | 0.01 |
|----|-------|------|
| 7: | ditto | 0.04 |
| 8: | ditto | 0.16 |
| 9: | ditto | 0.63 |
| 10: | ditto | 2.5 |
| 11: | ditto | 5.0 |
| 12: | ditto | 10.0 |

13: Molecular weight markers

# FIG. 2

←Position of objective band

1 2 3
Lane

1: Molecular weight markers
2: Method according to the invention
3: Conventional method

# FIG. 3A

←Position of objective band

1 2 3 4 5 6 7 8 9 10 11 12
Lane

Lanes 1–12: Plasma samples

# FIG. 3B

←Position of objective band

1 2 3 4 5 6 7 8 9 10 11 12
Lane

Lanes 1–12: Plasma samples

# FIG. 4

←—Position of objective band

1 2 3 4 5
Lane

1: Molecular weight markers
2: Water
3: Serum of normal person
4: Serum of HCV patient
5: Molecular weight markers

# FIG. 5

← 10 samples derived from nomal persons

← 10 samples derived from HCV patients

# FIG. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 428 197 (EASTMAN KODAK COMPANY)<br><br>* the whole document *<br>--- | 1-8, 11-15 | C12Q1/70<br>C12Q1/68<br>//C07H21/04 |
| P,Y | EP-A-0 435 229 (SANWA KANKYUSHO CO., LTD.)<br><br>* the whole document * | 1-8, 11-15 | |
| A | * claims 6-9 *<br>--- | 9,10 | |
| Y | EP-A-0 201 184 (CETUS CORPORATION)<br><br>* the whole document *<br>especially<br>* page 19 - page 20; example 10 *<br>--- | 6-8, 11-15 | |
| P,Y | EP-A-0 402 997 (AKZO N.V.)<br><br>* page 3, column 4, line 22 - line 43 *<br>--- | 1-4,6-8, 11-15 | |
| Y | PROCEEDINGS OF THE NATIONAL ACADEMY OF SCIENCES OF USA.<br>vol. 86, no. 1, January 1989, WASHINGTON US<br>pages 312 - 316;<br>S. KANEKO ET AL.: 'DETECTION OF SERUM HEPATITIS B VIRUS DNA IN PATIENTS WITH CHRONIC HEPATITIS USING THE POLYMERASE CHAIN REACTION ASSAY'<br>* the whole document *<br>--- | 1-4,6-8, 11-15 | TECHNICAL FIELDS SEARCHED (Int. Cl.5)<br><br>C12Q<br>C07K<br>C12N |
| Y | JAPANESE JOURNAL OF EXPERIMENTAL MEDICINE<br>vol. 60, no. 4, August 1990, TOKYO<br>pages 223 - 233;<br>H. OKAMOTO ET AL.: 'ENZYME-LINKED IMMUNOSORBENT ASSAY FOR ANTIBODIES AGAINST THE CAPSID PROTEIN OF HEPATITIS C VIRUS WITH A SYNTHETIC OLIGOPEPTIDE'<br>* page 224, right column, line 20 - line 23 *<br>----- | 1-8, 11-15 | |

The present search report has been drawn up for all claims

| Place of search | Date of completion of the search | Examiner |
|---|---|---|
| THE HAGUE | 10 MARCH 1992 | OSBORNE H.H. |